# EUROPEAN PATENT SPECIFICATION

(11) **EP 1 162 626 B1**
(45) Date of publication and mention of the grant of the patent: **02.06.2004**
(21) Application number: 00907976.5
(22) Date of filing: 09.03.2000
(51) Int. Cl.: G21G 4/04, G21G 4/08, A61K 51/00, A61N 5/10, A61K 51/12

(54) **RADIOACTIVE MICROSPHERE AND METHOD FOR PREPARATION THEREOF**
RADIOAKTIVE MIKROKUGEL UND HERSTELLUNGSVERFAHREN DAZU
MICROSPHERE RADIOACTIVE ET PROCEDE DE FABRICATION

(30) Priority: 11.03.1999 JP 6475899
(43) Date of publication of application: 12.12.2001
(73) Proprietor: Kansai Technology Licensing Organization Co., Ltd., Kyoto-shi, Kyoto 600-8815 (JP); Neturen Co., Ltd., Tokyo (JP); Shimizu-Tech Co., Ltd., Kobe-shi, Hyogo 651-2241 (JP)
(72) Inventor: KOKUBO, Tadashi, Nagaokakyo-shi, Kyoto 617-0841 (JP); KAWASHITA, Masakazu, Amagasaki-shi, Hyogo 661-0033 (JP); HIRAOKA, Masahiro, Sakyo-ku, Kyoto-shi, Kyoto 606-0033 (JP); NAGATA, Yasushi, Sakyo-ku, Kyoto-shi, Kyoto 606-8265 (JP); INOUE, Yoshiaki, Hiratsuka-shi, Kanagawa 254-0013 (JP); YAMAZAKI, Takao, Hiratsuka-shi, Kanagawa 254-0013 (JP); SHIMIZU, Yasuhiro, Nada-ku, Kobe-shi, Hyogo 657-0023 (JP); SAWADA, Yoshiki, Nada-ku, Kobe-shi, Hyogo 657-0054 (JP)
(74) Representative: Schirdewahn, Jürgen
(86) International application number: PCT/JP2000/001443
(87) International publication number: WO 2000/054284

(56) References cited:
- JP-A- 5 208 919
- JP-A- 7 222 804
- JP-A- 9 166 697
- JP-A- 62 501 076
- US-A- 3 697 435
- US-A- 4 789 501
- US-A- 4 889 707

## Description

The present invention relates to radioactive microspheres excellent in chemical durability, in particular microspheres favorably applicable for treating affected regions of a cancer patient by directly irradiating the regions with a radiation after injecting the radioactive microspheres into the patient's body.

The therapeutic methods for irradiating affected regions with a radiation for treating a cancer have an advantage that functional recoveries of organs can be expected over surgical methods by which the organs are excised. Among the methods described above, use of a therapeutic method for directly irradiating tumors with a radiation by injecting radioactive microspheres into the affected region through the blood vessel by means of a catheter is thought to be promising, since a sufficient dosage of radiation can be irradiated to the affected region without damaging normal tissues just under the surface of the body, as compared with a therapeutic method by which the radiation is irradiated from the outside of the body.

However, the radioactive microspheres with a too large particle size may be detained before they arrive at the affected region, while those with a too small particle size will penetrate through capillary blood vessels to fail in retaining them at the affected region. When the microspheres are readily dissolved in the body, on the other hand, radioactive elements are transferred to other portions in the body and normal cells are also damaged. A too long half-life also damages the normal tissues by emitting radioactive rays after the therapy, and a too short half-life causes a rapid decay of radioactivity to make the therapy unavailable. From these situations, the microspheres are required to have a diameter of 1 to 100 µm, preferably 20 to 30 µm, to be excellent in chemical durability, and to have a proper half-life.

Materials to be used for the conventional microspheres include an yttria-alumina-silica glass or a glass containing a small amount of phosphorous, wherein the non-radioactive element yttrium or phosphorous in the glass is converted into a β-emitter yttrium or phosphorous by irradiating slow neutrons (Japanese Patent Application Publication No. 6-62439). The glass named as YAS-4 in the patent publication and having a composition ratio of Y₂O₃ : Al₂O₃ : SiO₂ = 40 : 20 : 20 in % by weight (Y₂O₃ : Al₂O₃ : SiO₂ = 19 : 17 : 64 in mol%) has a most excellent chemical durability among the substances disclosed in the patent, and the microspheres having a proper particle size can be manufactured by a usual melting method.

Since β-emitter yttrium has a half-life as short as 64.1 hours, its radioactivity is remarkably decayed during transportation to a hospital after irradiating neutrons in a nuclear reactor. Accordingly, it is required for using the glass microspheres described in Japanese Patent Application Publication No. 6-62439 that the glass spheres contain as much yttria as possible.

However, the content of yttria is naturally restricted within a range capable of forming a glass, since the microspheres described in the patent publication is formed after manufacturing the glass. Since a large quantity of lactic acid is secreted at the tumors, pH in the vicinity of the microspheres is conjectured to be substantially low. The glass described in the patent publication above has a tendency that its chemical durability decreases as the content of yttria turns out to be high.

Phosphorous is also converted from a non-radioactive element to a β-emitter as in the case of yttrium by irradiating with slow neutrons. The β-emitter phosphorous has a considerably longer half-life of 14.3 days.

However, a glass containing a large quantity of phosphorous while being excellent in chemical durability has not been developed yet. Phosphorous in the currently available glass containing a large quantity of phosphorous is rapidly dissolved out of the glass in the body.

Accordingly, the object of the present invention is to provide microspheres being excellent in chemical durability, emitting a radiation for a long period of time in the body, and having a diameter of 1 to 100 µm.

Radioactive microspheres for attaining the object of the present invention comprise not less than 99% by weight of an oxide crystal containing 47% by weight or more of radioactive yttrium, and the balance of inevitable impurities.

The conventional yttria-alumina-silica glass (with a composition ratio of Y₂O₃ : Al₂O₃ : SiO₂ = 40 : 20 : 40 in % by weight) contains yttrium in a proportion of 31.5 % by weight (40 × 88.91 × 2/225.82). On the contrary, the radioactive microspheres according to the present invention having the composition as described above contain yttrium in a proportion of 47% × 0.99 = 46.5 % by weight or more, which is by 1.47 times as much as the yttrium content in the conventional glass. Accordingly, the remaining content of radioactive yttrium amounts 1.47 times as high as the radioactive yttrium in the conventional glass even after the half-life.

Since the weight fraction of yttrium in yttria is 88.91 × 2/225.82 when the oxide crystal is represented by Y₂O₃, the yttrium content is 78 % by weight or more, which is 2.47 times as high as the yttrium content in the conventional glass. Accordingly, a radiation with a dosage by 2.47 times as large as that of the conventional glass can be irradiated on the affected region.

The oxide crystal may be YPO₄, or a mixture of Y₂O₃ and YPO₄. While the lower limit of the yttrium content in the entire microspheres decreases to 48 % by weight when the oxide crystal is represented by YPO₄, the Y content remains to be higher than the conventional glass. Furthermore, since phosphorous having a substantially longer half-life is involved in place, radioactivity is additionally increases.

The radioactive microspheres according to the present invention may be coated with a film comprising at least one of the compounds selected from silica (SiO₂), titania (TiO₂), alumina (Al₂O₃), iron (III) oxide (Fe₂O₃), silicon nitride (Si₂N₃, SiN, Si₃N₄), aluminum nitride (AlN), titanium nitride (TiN), iron nitride (Fe₂N, Fe₄N), silicon carbide (SiC) and titanium carbide (TiC). Since these coating films do not emit harmful α- or γ-ray after irradiating with neutrons while having an acid resistant property, the radioactive elements are not dissolved in the strongly acidic body fluid at the tumors to enable a long irradiation of the tumor with the radiation. The preferable thickness of the coating film is 0.01 to 5 µm. When the thickness is less than 0.01 µm, erosion of acids is not effectively prevented while, when the thickness exceeds 5 µm, the proportion of yttrium in the entire microspheres becomes too small. Silica is the most preferable material for forming the coating film.

An appropriate method for manufacturing the microspheres according to the present invention is as follows.

At first, an oxide powder as a starting material containing yttrium, or yttrium and phosphorous, is melted by allowing the powder to pass through a thermal-plasma to obtain microspheres containing non-radioactive yttrium (or yttrium and phosphorous). The powder material can be melted, for example, by allowing it to pass through a thermal-plasma or laser beam. Yttria may be efficiently formed into spheres by the high temperature of the thermal-plasma, and phosphorous components in yttrium phosphate is not decomposed since the thermal-plasma is a heat source that does not use reactive gases such as oxygen and steam. However, the heating means are not restricted to the thermal-plasma and laser beam. Then, yttrium (or yttrium and phosphorous) is (are) turned into a radioactive element (elements) by irradiating with an effective dosage of neutrons. In order to enhance chemical durability, it is recommended to heat the microspheres in an oxidizing atmosphere before irradiating with the neutrons.

For forming the oxide coating film on the surface, the microspheres and a starting material of the coating film are placed in a plasma polymerization apparatus to form the coating film on the surface of the microspheres by plasma polymerization, followed by irradiating an effective dosage of neutrons to yttrium (or yttrium and phosphorous). Si, Ti, Al, Fe, O, N and C constituting the coating film are preferable as the coating film materials since they do not emit harmful radiation (α-ray and γ-ray) by being irradiated with neutrons. While plasma polymerization is one example of the methods for forming the coating film, other methods such as a sputtering method, a vacuum deposition method, a deposition method from solution, a chemical vapor deposition method, a molecular beam epitaxy method, ion-beam vapor deposition method and a sol-gel method may be used.
Fig. 1 describes a condition for forming the coating film on the substrate by plasma polymerization.
Fig. 2 shows a SEM photograph for observing the spherical shapes of the microspheres before and after forming the coating film.

### EXAMPLE 1

A fine powder containing 99.9 % by weight of yttria was melted with a high frequency induction thermal-plasma under the following condition to form into spheres.
Powder feed carrier gas: Ar 5L/min
Plasma gas composition: Ar 90 L/min + O₂ 5L/min
High frequency generator: anode input 40 kW, output frequency 4 MHz

A group of the microspheres were dispersed in ultra pure water with a specific conductance of 18 MΩ·cm, and classified with a nylon sieve to obtain microspheres containing not less than 99 % by weight of Y₂O₃ and having a diameter of 20 to 30 µm. The microspheres obtained were subjected to the following chemical durability test.

The microspheres (0.2g) were placed in a polypropylene bottle together with 20 ml of distilled water and, while maintaining the water temperature at 95°C in an oil bath, the bottle was shaken for 7 days with a stroke length of 3 cm at a speed of 120 strokes/min. After filtering the solution, the yttrium content in the filtrate was assayed by a high frequency inductively coupled plasma atomic emission spectrometry (ICP). The result showed that the proportion of dissolved yttrium is 1 ppm or less.

### COMPARATIVE EXAMPLE 1

The chemical durability test was carried out under the same condition as in Example 1, except that a glass with a composition ratio of Y₂O₃ : Al₂O₃ : SiO₂ = 40 : 20 : 20 was used for the radioactive microspheres, in place of the microspheres in Example 1. The result showed that the proportion of dissolved yttrium is 6 ppm.

A comparison of the result in Example 1 with the result in Comparative Example 1 shows that the microspheres comprising yttria according to the present invention are superior to the glass microspheres in chemical durability.

### EXAMPLE 2

Microspheres containing 99 % by weight or more of YPO₄ and having a diameter of 20 to 30 µm were manufactured by the same method as in Example 1 for manufacturing the yttria microspheres. These microspheres were heated at 900°C in the atmosphere for four hours.

The microspheres after heating were subjected to the same chemical durability test as in Example 1. The result showed that the proportion of dissolved yttrium and dissolved phosphorous were 1.2 ppm and 1.7 ppm, respectively. When the same chemical durability test was applied to the microspheres before heating in the atmosphere, the proportion of dissolved yttrium and dissolved phosphorous were 0.73 ppm and 85 ppm, respectively.

Accordingly, it was made clear that chemical durability is remarkably improved by heat treating the microspheres comprising the oxide crystal according to the present invention, even when the microspheres contain phosphorous that is liable to be readily dissolved out.

### EXAMPLE 3

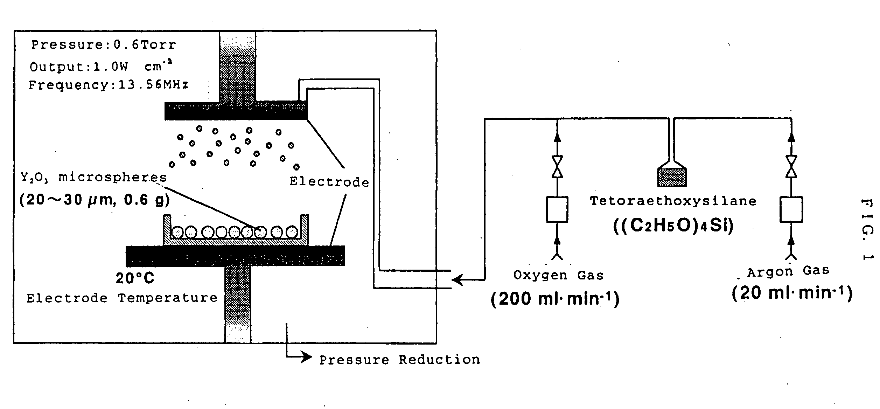

Microspheres (0.6g) obtained by the same manufacturing method as in Example 1 and containing 99 % by weight or more of Y₂O₃ with a diameter of 20 to 30 µm were placed in a polystyrene container with a size of 20 × 20 × 5 mm³. The container was placed in a plasma polymerization apparatus (Moldel BP-2, made by Samco International Research Laboratories, Co.) as shown in Fig. 1 to coat the surface of the microspheres with a SiO₂ film. Tetraethoxy silane (made by Shinetsu Chemical Industry Co.) was used as the starting material of SiO₂, oxygen was used as the reactive gas, and argon was used as a carrier gas. The conditions in this example were: inner pressure of the reaction chamber - 0.6 Torr; output - 1.0 w · cm⁻²; oxygen gas flow rate - 200 ml/min⁻¹, argon gas flow rate - 20 ml/min⁻¹; and electrode temperature - 20°C. For uniformly coating the entire surface of the microspheres, the film deposition time was adjusted to 15 minutes per one film deposition run, the polystyrene container was shaken after the first film deposition run, and the film deposition process was repeated again. The film deposition was repeated 4 times in total.

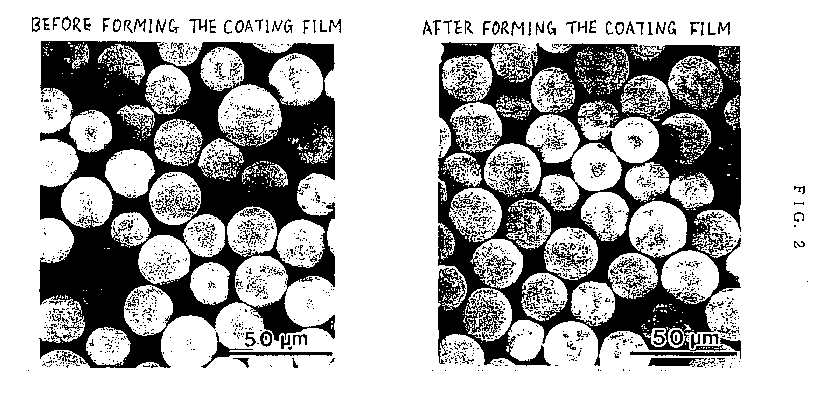

The microspheres coated with the SiO₂ film were observed under a scanning electron microscope (SEM, Model S-2500CX, made by Hitachi, Ltd.) to compare the coated microspheres with those before coating. The result in Fig. 2 showed that the shape was not changed even after coating, indicating that the coating film can be formed on the surface without changing the shape of the microspheres.

The microspheres with the coating film was subjected to the chemical durability test under the same condition as in Example 1, showing that the proportion of dissolved yttrium was 1 ppm or less.

### EXAMPLE 4

The chemical durability test was applied to the microspheres with the coating film in Example 3 by the same condition as in Example 3, except that a buffer solution with pH 4 (0.05 M potassium hydrogen phthalate (C₆H₄(COOK)(COOH)) was used in place of distilled water. The assay result showed that the proportion of dissolved yttrium was 3 ppm.

### REFERENCE EXAMPLE

The chemical durability test was applied to the microspheres used in Example 1 under the same condition as in Example 4 without forming the coating film. The result showed that the proportion of dissolved yttrium reaches 120 ppm.

### COMPARATIVE EXAMPLE 2

The chemical durability test was applied to the glass microspheres used in Comparative Example 1 under the same condition as used in Example 4. The assay result showed that the proportion of dissolved yttrium was 1.59 × 10³ ppm.

Comparisons of the results in Examples 3 and 4 with the results in Reference Example and Comparative Example 2 showed that acid resistance remarkably increases by forming the coating film on the surface of the microspheres.

The radioactive microspheres of the present invention as hitherto described contains a high concentration of radioactive elements that are hardly dissolved. Accordingly, the microspheres are useful as a medical material for treating tumors by direct irradiation of radiation by embedding the microspheres in the vicinity of the tumors.

## Claims

1. A radioactive microsphere comprising not less than 99% by weight of an oxide crystal containing 47% by weight or more of radioactive yttrium, and the balance of inevitable impurities.

2. The radioactive microsphere according to claim 1, wherein the oxide crystal consists essentially of Y₂O₃.

3. The radioactive microsphere according to claim 1, wherein the oxide crystal consists essentially of YPO₄, or a mixture of Y₂O₃ and YPO₄.

4. The radioactive microsphere according to claim 1, wherein the microsphere has a diameter of 1 to 100 µm.

5. The radioactive microsphere according to claim 1, wherein the microsphere has a diameter of 20 to 30 µm.

6. The radioactive microsphere according to any one of claims 1 to 5, wherein the microsphere is coated with a film comprising at least one of the compounds selected from silica (SiO₂), titania (TiO₂), alumina (Al₂O₃), iron (III) oxide (Fe₂O₃), silicon nitride (Si₂N₃, SiN, Si₃N₄), aluminum nitride (AlN), titanium nitride (TiN), iron nitride (Fe₂N, Fe₄N), silicon carbide (SiC) and titanium carbide (TiC).

7. The radioactive microsphere according to claim 6, wherein the film has a thickness of 0.01 to 5 µm.

8. A method of producing a radioactive microsphere, the method comprising preparing a microsphere comprising not less than 99% by weight of an oxide crystal containing 47% by weight or more of non-radioactive yttrium, and the balance of inevitable impurities through melting of a starting material, followed by irradiating with an effective dosage of slow neutrons to turn non-radioactive yttrium into a radioactive element.

9. A method of producing a radioactive microsphere, the method comprising preparing a microsphere comprising not less than 99% by weight of an oxide crystal containing 47% by weight or more of non-radioactive yttrium and an amount of phosphrous, and the balance of inevitable impurities through melting of a starting material, followed by heating the microsphere in an oxidizing atmosphere and then irradiating with an effective dosage of slow neutrons to turn non-radioactive yttrium into a radioactive element.

10. The method according to claim 8 or 9, further comprising coating the microsphere with a film after preparing the microsphere or heating in the oxidizing atmosphere and before irradiating with an effective dosage of slow neutrons, the film comprising at least one of the compounds selected from silica (SiO₂), titania (TiO₂), alumina (Al₂O₃), iron (III) oxide (Fe₂O₃), silicon nitride (Si₂N₃, SiN, Si₃N₄), aluminum nitride (AlN), titanium nitride (TiN), iron nitride (Fe₂N, Fe₄N), silicon carbide (SiC) and titanium carbide (TiC).

## Patentansprüche

1. Radioaktive Mikrokugel umfassend mindestens 99 Gewichtsprozent einen Oxidkristall, der mindestens 47 Gewichtsprozent an radioaktivem Yttrium enthält, sowie einen Rest unvermeidbarer Verunreinigungen.

2. Radioaktive Mikrokugel gemäß Anspruch 1, wobei der Oxidkristall aus Y₂O₃ besteht.

3. Radioaktive Mikrokugel gemäß Anspruch 1, wobei der Oxidkristall aus YPO₄ oder einer Mischung aus Y₂O₃ und YPO₄ besteht.

4. Radioaktive Mikrokugel gemäß Anspruch 1, wobei die Mikrokugel einen Durchmesser von 1 bis 100 µm aufweist.

5. Radioaktive Mikrokugel gemäß Anspruch 1, wobei die Mikrokugel einen Durchmesser von 20 bis 30 µm aufweist.

6. Radioaktive Mikrokugel gemäß einem der Ansprüche 1 bis 5, wobei die Mikrokugel mit einem Film beschichtet ist, der wenigstens eine der folgenden Verbindungen umfasst: Siliziumdioxid (SiO₂), Titandioxid (TiO₂), Aluminiumoxid (Al₂O₃), Eisen(III)-oxid (Fe₂O₃), Siliziumnitrid (Si₂N₃, SiN, Si₃N₄), Aluminiumnitrid (AIN), Titannitrid (TIN), Eisennitrid (Fe₂N, Fe₄N), Siliziumcarbid (SiC) und Titancarbid (TiC).

7. Radioaktive Mikrokugel gemäß Anspruch 6, wobei der Film eine Dicke von 0,01 bis 5 µm aufweist.

8. Verfahren zur Herstellung einer radioaktiven Mikrokugel, umfassend folgende Schritte: Herstellung einer Mikrokugel, umfassend mindestens 99 Gewichtsprozent einen Oxidkristall, der mindestens 47 Gewichtsprozent an nicht radioaktivem Yttrium enthält, sowie einen Rest unvermeidbarer Verunreinigungen, durch Schmelzen eines Ausgangsmaterials gefolgt von der Bestrahlung mit einer effektiven Dosis langsamer Neutronen, um das nicht radioaktive Yttrium in ein radioaktives Element umzuwandeln.

9. Verfahren zur Herstellung einer radioaktiven Mikrokugel, umfassend folgende Schritte: Herstellung einer Mikrokugel, umfassend mindestens 99 Gewichtsprozent einen Oxidkristall, der mindestens 47 Gewichtsprozent an nicht radioaktivem Yttrium und einen Anteil Phosphor enthält, sowie einen Rest unvermeidbarer Verunreinigungen, durch Schmelzen eines Ausgangsmaterials gefolgt vom Erhitzen der Mikrokugel in einer oxidierenden Atmosphäre und danach der Bestrahlung mit einer effektiven Dosis langsamer Neutronen, um das nicht radioaktive Yttrium in ein radioaktives Element umzuwandeln.

10. Verfahren gemäß Anspruch 8 oder 9 weiter umfassend das Beschichten der Mikrokugel mit einem Film nach der Herstellung der Mikrokugel bzw. nach dem Erhitzen in der oxidierenden Atmosphäre und vor der Bestrahlung mit einer effektiven Dosis langsamer Neutronen, wobei der Film wenigstens eine der folgenden Verbindungen umfasst: Siliziumdioxid (SiO₂), Titandioxid (TiO₂), Aluminiumoxid (Al₂O₃), Eisen(III)-oxid (Fe₂O₃), Siliziumnitrid (Si₂N₃, SiN, Si₃N₄), Aluminiumnitrid (AIN), Titannitrid (TIN), Eisennitrid (Fe₂N, Fe₄N), Siliziumcarbid (SiC) und Titancarbid (TiC).

## Revendications

1. Microsphère radioactive comprenant pas moins de 99 % en poids d'un cristal d'oxyde contenant 47 % en poids
ou plus d'yttrium radioactif, et le reste étant constitué d'inévitables impuretés.

2. Microsphère radioactive selon la revendication 1, dans laquelle le cristal d'oxyde est pratiquement composé de Y₂O₃.

3. Microsphère radioactive selon la revendication 1, dans laquelle le cristal d'oxyde est pratiquement composé de YPO₄ ou d'un mélange de Y₂O₃ et de YPO₄.

4. Microsphère radioactive selon la revendication 1, dans laquelle la microsphère présente un diamètre de 1 à 100 µm.

5. Microsphère radioactive selon la revendication 1, dans laquelle la microsphère présente un diamètre de 20 à 30 µm.

6. Microsphère radioactive selon l'une quelconque des revendications 1 à 5, dans laquelle la microsphère est revêtue d'un film comprenant au moins un des composés choisis parmi la silice (SiO₂), le dioxyde de titane (TiO₂), l'alumine (Al₂O₃), l'oxyde de fer (III) (Fe₂O₃), les nitrures de silicium (Si₂N₃, SiN, Si₃N₄), le nitrure d'aluminium (AlN), le nitrure de titane (TiN), les nitrures de fer (Fe₂N, Fe₄N), le carbure de silicium (SiC) et le carbure de titane (TiC).

7. Microsphère radioactive selon la revendication 6, dans laquelle le film a une épaisseur de 0,01 à 5 µm.

8. Procédé de fabrication d'une microsphère radioactive, le procédé comprenant la préparation d'une microsphère comprenant pas moins de 99 % en poids d'un cristal d'oxyde contenant 47 % ou plus d'yttrium non radioactif, et le reste d'inévitables impuretés, par fusion d'un matériau de départ, suivie par une irradiation au moyen d'un dosage efficace de neutrons lents afin de transformer l'yttrium non radioactif en un élément radioactif.

9. Procédé de fabrication d'une microsphère radioactive, le procédé comprenant la préparation d'une microsphère comprenant pas moins de 99 % en poids d'un cristal d'oxyde contenant 47 % ou plus d'yttrium non radioactif et une quantité de phosphore, et le reste d'inévitables impuretés, par fusion d'un matériau de départ, suivie par le chauffage de la microsphère dans une atmosphère oxydante, puis par une irradiation au moyen d'un dosage efficace de neutrons lents afin de transformer l'yttrium non radioactif en un élément radioactif.

10. Procédé selon la revendication 8 ou 9, comprenant en outre une étape consistant à revêtir la microsphère par un film après la préparation de la microsphère ou le chauffage dans une atmosphère oxydante, et avant l'irradiation au moyen d'une dose efficace de neutrons lents, le film comprenant au moins un des composés choisi parmi la silice (SiO₂), le dioxyde de titane (TiO₂), l'alumine (Al₂O₃), l'oxyde de fer (III) (Fe₂O₃), les nitrures de silicium (Si₂N₃, SiN, Si₃N₄), le nitrure d'aluminium (AIN), le nitrure de titane (TiN) , les nitrures de fer (Fe₂N, Fe₄N), le carbure de silicium (SiC) et le carbure de titane (TiC).
